# EUROPEAN PATENT SPECIFICATION

(11) **EP 3 515 960 B1**
(45) Date of publication and mention of the grant of the patent: **01.06.2022**
(21) Application number: 17755634.7
(22) Date of filing: 18.08.2017
(51) Int. Cl.: C08G 18/61, A61Q 5/12, A61Q 5/02, A61Q 1/02, A61K 8/898, C09D 175/16, A61F 13/00, A61F 13/02, A61K 9/70, C08G 18/18, C08G 18/24, C08G 18/28, C08G 18/40, C08G 18/42, C08G 18/44, C08G 18/48, C08G 18/67, C08G 18/73, C08G 18/75, C08G 18/81, C09J 175/16

(54) **PERSONAL CARE COMPOSITIONS INCLUDING A POLYURETHANE-POLYORGANOSILOXANE COPOLYMER**
KÖRPERPFLEGEZUSAMMENSETZUNGEN MIT POLYURETHAN-POLYORGANOSILOXANCOPOLYMER
COMPOSITIONS DE SOINS PERSONNELS COMPRENANT UN COPOLYMÈRE POLYURÉTHANE-POLYORGANOSILOXANE

(30) Priority: 19.09.2016 US 201662396328 P; 19.09.2016 US 201662396323 P; 15.02.2017 EP 17305166
(43) Date of publication of application: 31.07.2019
(73) Proprietor: Dow Silicones Corporation, Midland, MI 48686-0994 (US); Dow France S.A.S., 93200 Saint Denis (FR)
(72) Inventor: ZHU, Bizhong, Midland MI 48640 (US); GRASMANN, Martin, Midland MI 48640 (US); PANDIT, Vinita, Midland MI 48642 (US); THOMAS, Xavier, 59300 Famars (FR)
(74) Representative: Murgitroyd & Company
(86) International application number: PCT/US2017/047469
(87) International publication number: WO 2018/052647

(56) References cited:
- EP-A1- 1 588 686
- WO-A1-2016/084890
- US-A- 5 075 399
- US-A- 5 643 581
- US-A1- 2006 142 526
- US-A1- 2006 247 403
- US-A1- 2007 071 700
- US-A1- 2007 154 440
- US-B1- 6 524 564
- DATABASE WPI Week 201319 Thomson Scientific, London, GB; AN 2013-B37881 XP002775811, -& CN 102 757 705 A (ZHANG B) 31 October 2012 (2012-10-31)

## Description

### TECHNICAL FIELD

A polyurethane - polyorganosiloxane copolymer is useful in personal care compositions, such as hair care compositions and skin care compositions.

### BACKGROUND

Silicone resins (such as MQ and MQ-T type resins) have been used as film forming agents in personal care compositions. These silicone resins may suffer from the drawback of providing insufficient abrasion resistance (resistance to wear off) to the personal care compositions, or in some other instances may be too brittle and develop cracks relatively easily. Polyurethanes are being used also for these applications. While they have better abrasion resistance they can suffer from inferior water resistance and other disadvantages as compared with silicone resins. There is an industry need for improved film forming agents for use in personal care compositions. US2007/154440 discloses long wearing cosmetic compositions comprising high molecular weight polyurethane polymers. The polyurethane polymers typically will have a molecular weight greater than about 50,000.

### SUMMARY

This invention relates to a composition comprising a polyurethane - polyorganosiloxane copolymer of formula I or II and a carrier. The carrier can permit application to substrates such as skin, leather, hair, textiles, and/or other fibers. This invention also provides a method of using the polyurethane - polyorganosiloxane copolymer of formula I or II for treatment of such substrates. This invention further provides a method for using the the polyurethane - polyorganosiloxane copolymer of formula I or II as a film forming agent. Particular embodiments of the invention are set forth in the dependent claims. Associated methods are also described herein to aid in the understanding of the invention, but these do not form part of the claimed invention. Examples or embodiments described herein which do not fall under the definition of the claims do not form part of the present invention.

### DETAILED DESCRIPTION

The present invention relates to a personal care composition comprising:
(1) a polyurethane - polyorganosiloxane copolymer of the formula (I): 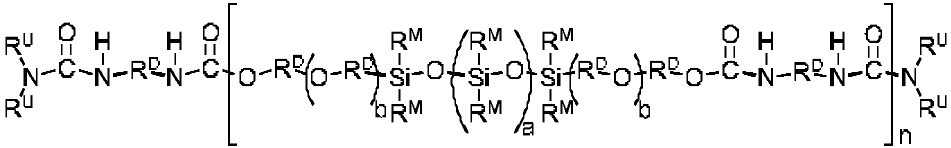 where each R^{U} is independently an alkenyl group of 2 to 13 carbon atoms; each R^{D} is independently an alkylene group of 2 to 13 carbon atoms; each R^{M} is independently a monovalent hydrocarbon group or a monovalent halogenated hydrocarbon group; each subscript b is independently 0 or 1; each subscript a is independently 0 to 100,000; and subscript n is 1 to 10,000; and (2) a carrier that permits application.

Each R^{U} may be independently selected from alkenyl groups such as vinyl, allyl, butenyl, or hexenyl; alternatively vinyl or allyl.

Each R^{D} may be an alkylene group such as ethylene or propylene.

Each R^{M} may have 1 to 13 carbon atoms. Alternatively, each R^{M} may be a monovalent hydrocarbon group free of aliphatic unsaturation. For example, each R^{M} may be independently selected from alkyl such as methyl, ethyl, propyl, butyl or hexyl; aryl such as phenyl, or aralkyl such as tolyl, xylyl or phenyl-methyl. Alternatively, each R^{M} may be methyl or phenyl, and alternatively each R^{M} may be methyl.

Alternatively, subscript a is 0 to 50,000, 0 to 10,000, 0 to 5,000, 0 to 1,000, 1 to 1,000, 1 to 500, 1 to 200, or 5 to 150. Alternatively, subscript n is 1 to 5,000, 1 to 1,000, 1 to 500, or 1 to 200.

The invention also relates to a personal care composition comprising a polyurethane - polyorganosiloxane copolymer of formula (II): 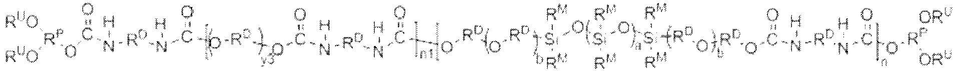 where
each R^{U} is independently an alkenyl group of 2 to 13 carbon atoms;
each R^{D} is independently an alkylene group of 2 to 13 carbon atoms;
each R^{M} is independently a monovalent hydrocarbon group or a monovalent halogenated hydrocarbon group;
each R^{P} is independently a trivalent hydrocarbon group or a trivalent halogenated hydrocarbon group;
each subscript b is independently 0 or 1;
each subscript a is independently 0 to 100,000; and
subscript n is 1 to 10,000
each subscript y3 is independently 1 to 200,000; and
subscript n1 is 1 to 1,500,000.
Alternatively, subscript y3 is 1 to 100,000, 1 to 50,000, 1 to 10,000, 1 to 5,000, 1 to 1,000, 1 to 500, 1 to 100., 1 to 100, 1 to 50, or 1 to 20. Alternatively, subscript y3 = 2, 5, 9, 20, or 40. Alternatively, subscript n1 is 1 to 200,000, 1 to 50,000, 1 to 10,000, 1 to 5,000, 1 to 1,000, 1 to 500 or 1 to 200.

The polyurethane - polyorganosiloxane copolymer described above may be prepared by a method (not claimed) comprising:
i) reacting starting materials comprising:
   a) an isocyanate compound, and
   b) a polyorganosiloxane, thereby preparing a polyurethane - polyorganosiloxane prepolymer; and
ii) reacting the prepolymer prepared in step i) with a starting material comprising c) an endblocker having an average of one or more aliphatically unsaturated group per molecule; thereby preparing the polyurethane - polyorganosiloxane copolymer.

Alternatively, the polyurethane - polyorganosiloxane copolymer may be prepared by a method (not claimed) comprising:
i) reacting starting materials comprising:
   a) an isocyanate compound, and
   c) an endblocker having an average of one or more aliphatically unsaturated group per molecule, thereby preparing a urethane functional intermediate; and
ii) reacting the polyurethane functional intermediate prepared in step i) with a starting material comprising b) a polyorganosiloxane; thereby preparing the polyurethane - polyorganosiloxane copolymer.

In each of the unclaimed copolymer preparation methods described above, b) the polyorganosiloxane may be b1) a carbinol terminated polyorganosiloxane, b2) an amine terminated polyorganosiloxane, or a mixture of both b1) and b2).

Alternatively, in the said copolymer preparation methods described above, d) a chain extender may optionally be added as a starting material in addition to a) the isocyanate, b) the polyorganosiloxane, and c) the endblocker. Starting material d), the chain extender, may be added before, during, and/or after step i) in each of the methods described above. In one example, b) the polyorganosiloxane may be pre-reacted with d) the chain extender before reacting b) the polyorganosiloxane in the method. Alternatively, a) the isocyanate compound may be pre-reacted with d) the chain extender before reacting the a) isocyanate compound in the method.

The isocyanate compound is exemplified by monomeric isocyanates such as vinyl isocyanate. The isocyanate group can be blocked by common blocking agents such as phenol, nonyl phenol, butanone oxime, caprolactam, and others. These blocked isocyanates can be released by any conventional means such as heating at a temperature above room temperature to react with chain extenders and polyorganosiloxanes to construct the polyurethane - polyorganosiloxane copolymer.

In the method described above, b1) the carbinol-functional polyorganosiloxane comprises units of formulae: 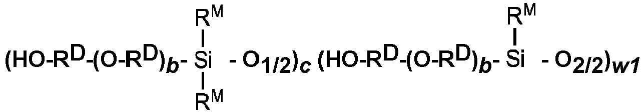 **(HO-R^{D}-(O-R^{D} )*_{b}*- Si** - **O_{3/2})*_{w}*(R^{M}₂SiO)*_{d}*(R^{M}SiO_{3/2})*ₑ*(SiO_{4/2})*ₕ*.** In this unit formula, each R^{M}, R^{D} and subscript b are described above. Subscript c, subscript w1, subscript w3, subscript d, subscript e, and subscript h are as follows: Subscript c ≥ 0. Alternatively, subscript c is 0 to 200,000; alternatively 0 to 100,000; alternatively 0 to 50,000; alternatively 0 to 10,000; alternatively 0 to 5,000; alternatively 0 to 1,000; alternatively 0 to 500; alternatively 0 to 100; alternatively 0 to 50; alternatively 0 to 20; and alternatively 0 to 10. Alternatively, subscript c is 1 to 100; alternatively 1 to 50; alternatively 1 to 20; and alternatively 1 to 10.

Subscript w1 ≥ 0. Alternatively, subscript w1 is 0 to 200,000; alternatively 0 to 50,000; alternatively 0 to 10,000; alternatively 0 to 5,000; alternatively w1 is 0 to 1,000; alternatively 0 to 500; alternatively 0 to 100; alternatively 0 to 50; alternatively 0 to 20; and alternatively 0 to 10. Alternatively, subscript w1 is 1 to 100; alternatively 1 to 50; alternatively 1 to 20; and alternatively 1 to 10.

Subscript w3 ≥ 0. Alternatively, subscript w3 is 0 to 200,000; alternatively 0 to 50,000; alternatively 0 to 10,000; alternatively 0 to 5,000; alternatively 0 to 1,000; alternatively 0 to 500; alternatively 0 to 100; alternatively 0 to 50; alternatively 0 to 20; and alternatively 0 to 10. Alternatively, subscript w3 is 1 to 100; alternatively 1 to 50; alternatively 1 to 20; and alternatively 1 to 10.

Subscripts d, e, and h depend on the molecular weight of one of the siloxane segments in the copolymer and may be without limit (e.g., bound only by the molecular weights reachable by the state of the art of siloxane synthesis chemistry). However, subscript d may be 0 to 1,000,000; subscript e may be 0 to 1,000,000; subscript h may be 0 to 1,000,000, and with the proviso that a quantity (d+e+h) ≥1. Subscript d ≥ 0. Alternatively, subscript d > 0. Alternatively, subscript d is 0 to 200,000, alternatively 0 to 100,000, alternatively 0 to 50,000, alternatively 0 to 10,000, alternatively 0 to 5,000, alternatively 0 to 1,000, alternatively 1 to 1,000, alternatively 1 to 500, and alternatively 1 to 200.

Subscript e ≥ 0. Alternatively, subscript e is 0 to 1,000,000. Alternatively, subscript e is 0 to 200,000; alternatively 0 to 100,000; alternatively 0 to 50,000; alternatively 0 to 10,000; alternatively 0 to 5,000; and alternatively 0 to 1,000. Alternatively, subscript e is 1 to 1,000; alternatively 1 to 500; and alternatively 1 to 200. Alternatively, subscript e = 0.

Subscript h is ≥ 0. Alternatively, subscript h is 0 to 1,000,000; alternatively 0 to 200,000; alternatively 0 to 100,000; alternatively 0 to 50,000; alternatively 0 to 10,000; alternatively 0 to 5,000; and alternatively 0 to 1,000. Alternatively, subscript h is 1 to 1,000; alternatively 1 to 500; and alternatively 1 to 200. Alternatively, subscript h = 0. Examples of carbinol-functional polyorganosiloxanes are disclosed in WO2008/088491, U.S. Patent 6,528,121, and U.S. Patent 7,452,956. The carbinol groups may be at terminal positions, pendent positions, or both terminal and pendent positions in the carbinol-functional polyorganosiloxane. Alternatively, the carbinol groups may be at terminal positions.

An exemplary amine terminated polyorganosiloxane comprises a terminal unit of formula 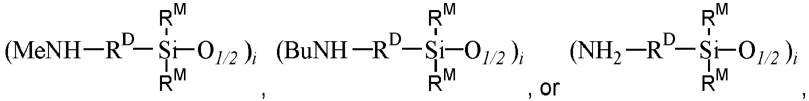 where Me represents a methyl group and Bu represents a butyl group; and further comprises one or more of (R^{M}₂SiO_{2/2})*_{d}*(R^{M}SiO_{3/2})*ₑ*(SiO_{4/2})*ₕ*, where R^{M}, R^{D}, and subscripts , d, e, and h are as described above.

The endblocker is a compound having an average of more than one aliphatically unsaturated group per molecule, and said compound is selected from an amine compound, an alcohol, or a thiol compound. The endblocker may be selected from compounds of formulae: 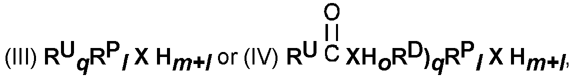 or mixtures of compounds (III) and (IV), where R^{U}, R^{P}, R^{D}, are as described above and X and subscripts *q*, *l, m,* and *o are* as *follows:* Subscript q indicates the number of aliphatically unsaturated hydrocarbon groups at a terminus of the polymer. In the formula above, 0 < q ≤ 3. Alternatively, 1 < q ≤ 3, alternatively, 1 ≤ q ≤ 3, and alternatively 2 ≤ q ≤ 3. Each X is independently nitrogen (N), oxygen (O), or sulfur (S). Alternatively, X is N or O. Alternatively, each X is N. Alternatively, each X is O. Subscript m = 1 when X is N, and subscript I = 1. Subscript m = 0 when X is O or S. When X is nitrogen, and subscript I = 0; then subscript m is 0. Subscript I is 0 or 1 when X is N, and subscript I = 1 when X is O or S. Subscript o = 0 when X is O or S, and subscript o = 1 when X is N.. The endblocker is added in an amount sufficient to provide a molar ratio of endblocker to isocyante (XH/N=C=O) < 1.

Alternatively, the endblocker may be an amine compound. The amine compound may have formula R^{U}_{z}N(R^{D})_{2-z}H, where R^{U} is as described above, and subscript z > 0, alternatively 0 < z ≤ 2, and alternatively z = 2. Examples of suitable amine compounds for the endblocker include diethyl amine and diallyl amine.

The chain extender may be a dialcohol, of formula HO-R^{D}-OH, a dihydroxyl compound, or R^{T}_{z1} NH_{2-z1}-R^{D}-NH_{2-z1} R^{T}_{z1,} a diamine, where R^{D} is as defined above and R^{T} is hydrogen or a monovalent hydrocarbon group. z1 is 0 or 1. The monovalent hydrocarbon group for R^{T} may have 1 to 13 carbon atoms. The monovalent hydrocarbon group for R^{T} is group independently selected from alkyl such as methyl, ethyl, propyl, butyl, or hexyl; aryl such as phenyl; or aralkyl such as tolyl, xylyl, or phenyl-methyl. Alternatively, each R^{T} may be methyl or phenyl. Alternatively each R^{T} may be hydrogen or methyl., Suitable dialcohols include 1,3-butanediol; 1,4-butanediol; 1,6-hexanediol, 1,10-decanediol; 1,6-hexamethylenediol; 2,2-dimethyl-1,3-propanediol; 1,4-cyclohexanedimethylol; 1,1'-isopropylidine-bis-(p-phenylene-oxy)-di-2-ethanol; poly(tetrmethylene ether) glycol; and ethylene glycol. Alternatively, the chain extender may be a diamine containing 2 to 20 carbon atoms e.g., 1,2-diaminoethane; 1,4-diaminobutane; 1,2-propanediamine; hexamethylenediamine; diethylene diamine; 5-amino-1-(aminomethyl)-1,3,3-trimethylcyclohexane; 4,4'-methylene bis(cyclohexylamine); and ethanol amine. Alternatively, the chain extender may be a dithiol, a dicarboxylic acid, or a diepoxide. Suitable chain extenders are disclosed, for example, in U.S. Patents 4,840,796 and 5,756,572.

After the reaction step ii) as described above, optionally the reaction product can be treated with an additional end blocker. This additional end blocker, e), can be such that it leaves an additional reactive group on the copolymer after end blocking reaction, or it leaves an unreactive group on the copolymer after the end blocking reaction. Suitable such end blockers for starting material e) include but are not limited to alcohols such ethanol, propanol, butanol, carboxylic acids such as acetic acid, and alcohols and carboxylic acids containing aliphatic unsaturation. Thio-alcohols, hydroxylamines, glycol, amino acids, and amino sugars are also suitable as additional endblocking agents. When isocyanate is present in molar excess during preparation of the copolymer, unreacted isocyanate can be present in the copolymer. Starting material e), the additional endblocker may be added to react with this residual isocyanate.

A solvent may be added during the method to prepare the polyurethane - polyorganosiloxane copolymer described herein. Any organic compound that will dissolve the polyurethane - polyorganosiloxane copolymer and that is relatively unreactive towards isocyanate, and amine and/or carbinol compounds is suitable as a solvent. Examples include aliphatic hydrocarbons, aromatic hydrocarbons, esters, ethers, ketones, and amides. Exemplary solvents include ethyl acetate, butyl acetate, methyl ethyl ketone, or tetrahydrofuran.

The amount of solvent to be used depends on the properties of the polyurethane - polyorganosiloxane copolymer including structure, molecular weight, and the particular method of copolymer preparation, and can be 0 to 99%. Generally for higher molecular weight copolymers especially when a high torque mixing mechanim will not be used, solvent may be added to reduce the viscosity and make the system easier to handle during performance of the method to make the polyurethane - polyorganosiloxane copolymer. If the molecular weight is relatively low and/or high torque mixing equipment such as a twin screw extruder is used, no solvent needs to be used. When solvent is used, the amount may be 0 to 99%, alternatively 0 to 80%, alternatively 1% to 60%, and alternatively 5% to 50%, based on the combined weights of all starting materials used.

Reacting b) the polyorganosiloxane with either the isocyanate compound or the isocyanate functional urea intermediate may be catalyzed by starting material g) a catalyst. Suitable catalysts include tertiary amines and metal salts, such as the salts of tin. Tin compounds are useful as catalysts herein include those where the oxidation state of the tin is either +4 or +2, i.e., tin (IV) compounds or tin (II) compounds. Examples of tin (IV) compounds include stannic salts such as dibutyl tin dilaurate, dimethyl tin dilaurate, di-(n-butyl)tin bis-ketonate, dibutyl tin diacetate, dibutyl tin maleate, dibutyl tin diacetylacetonate, dibutyl tin dimethoxide, carbomethoxyphenyl tin tris-uberate, dibutyl tin dioctanoate, dibutyl tin diformate, isobutyl tin triceroate, dimethyl tin dibutyrate, dimethyl tin di-neodecanoate, dibutyl tin di-neodecanoate, triethyl tin tartrate, dibutyl tin dibenzoate, butyltintri-2-ethylhexanoate, dioctyl tin diacetate, tin octylate, tin oleate, tin butyrate, tin naphthenate, dimethyl tin dichloride, a combination thereof, and/or a partial hydrolysis product thereof. Tin (IV) compounds are known in the art and are commercially available, such as Metatin^{®} 740 and Fascat^{®} 4202 from Acima Specialty Chemicals of Switzerland, Europe, which is a business unit of The Dow Chemical Company. Examples of tin (II) compounds include tin (II) salts of organic carboxylic acids such as tin (II) diacetate, tin (II) dioctanoate, tin (II) diethylhexanoate, tin (II) dilaurate, stannous salts of carboxylic acids such as stannous octoate, stannous oleate, stannous acetate, stannous laurate, stannous stearate, stannous naphthanate, stannous hexanoate, stannous succinate, stannous caprylate, and a combination thereof. Other metal salts are also suitable catalysts for this reaction. Examples include zinc salts such as zinc acetate and zinc naphthenate. Salts of lead, bismuth, cobalt, iron, antimony, and sodium, such as lead octoate, bismuth nitrate, and sodium acetate can also catalyze this reaction. In certain occasions organomercuric compounds can also be used. Optionally co-catalysts can also be used along with a primary catalyst. And a combination of two or more catalysts can be used, e.g., to provide either faster reaction than achievable with a single catalyst, or a better balanced reaction initiation time and finish time.

Starting material h) is an organic polyol. Suitable organic polyols are organic polymers containing two or more hydroxyl groups. The organic polyol for starting material h) may be a polyether polyol, a polyester polyol, a polyacrylate polyol, a polycaprolactone polyol, a polyurethane polyol, a polycarbonate polyol, polybutadiene diol, other polymer polyols, or two or more of these organic polyols. Copolymer polyols of two or more types of polymers can also be used. Polyols with other modifications on the polymer structures, such as fluorination, can also be used. Suitable organic polyols alternatively may be an organic polymer diol. Such organic polymer diols include polyalkylene oxide diols e.g., polyethylene oxide diols, polypropylene oxide diols, and polybutylene oxide diols; or polycarbonate diols. Suitable organic polyols alternatively may be small molecule organic diols. Such small molecule organic diols include glycerol. The organic polyol may be added to tune the surface energy and/or hydrophilicity/mechanical properties of the copolymer composition. The amount added may be 0 to 95%, alternatively 0 to 75%, alternatively 0 to 50%, and alternatively 1 to 25%.

The amounts of starting materials a), b), c), and when present one or more of, d), e), f), g), and h), can vary widely, according to the polyorganosiloxane structure and molecular weight desired, to arrive at the polyurethane - polyorganosiloxane copolymer described by the formula (I) or (II) defined herein. The molar ratio of isocyanate groups of starting material a) to the active hydrogen of carbinol or amine groups on the polysiloxane selected for starting material b) can be 0.1 to 100, alternatively 0.1 to 50, alternatively 0.1 to 10, alternatively 0.1 to 2, alternatively 0.1 to 1.5, alternatively 0.1 to 1.25, alternatively 0.1 to 1.1, alternatively 0.1 to 1.05, alternatively 0.1 to 1.01, alternatively 0.1 to 1, alternatively 0.1 to 0.9, alternatively 0.1 to 0.5, alternatively 0.5 to 50, alternatively 0.5 to 10, alternatively 0.5 to 2, alternatively 0.5 to 1.5, alternatively 0.5 to 1.25, alternatively 0.5 to 1.1, alternatively 0.5 to 1.05, alternatively 0.5 to 1.01, alternatively 0.5 to 1, alternatively 0.5 to 0.9, and alternatively 0.4 to 0.7. When this ratio is < 1, the reaction is controlled so that the endblocker is added before all the isocyanate groups are consumed. When this ratio is > 1, the endblocker can be added before or after all the active hydrogen on the carbinol or amine groups have been reacted. The molar ratio between the endblocker to the isocyanate can be from 0.001 to 0.99, alternatively 0.001 to 0.8, alternatively 0.01 to 0.8, alternatively 0.01 to 0.6, alternatively 0.01 to 0.5, alternatively 0.01 to 0.4, alternatively 0.01 to 0.3, alternatively 0.01 to 0.2, alternatively 0.01 to 0.1, alternatively 0.05 to 0.8, alternatively 0.05 to 0.6, alternatively 0.05 to 0.5, alternatively 0.05 to 0.4, alternatively 0.05 to 0.3, alternatively 0.05 to 0.2, alternatively 0.05 to 0.1. The molar ratio between the isocyante groups to the active hydrogen on the hydroxyl or amine groups or other reactive groups on the chain extender can be 1.001 to 1,000,000, alternatively 1.001 to 500,000, alternatively 1.001 to 200,000, alternatively 1.001 to 100,000, alternatively 1.001 to 50,000, alternatively 1.001 to 10,000, alternatively 1.001 to 5,000, alternatively 1.001 to 1,000, alternatively 1.001 to 500, alternatively 1.001 to 100, alternatively 1.001 to 50, alternatively 1.001 to 20, alternatively 1.001 to 10, alternatively 1.001 to 5, alternatively 1.001 to 4, alternatively 1.001 to 3, alternatively 1.001 to 2, alternatively 1.001 to 1.5, alternatively 1.001 to 1.3, alternatively 1.001 to 1.2, alternatively 1.01 to 20, alternatively 1.01 to 10, alternatively 1.01 to 5, alternatively 1.01 to 4, alternatively 1.01 to 3, alternatively 1.01 to 2, alternatively 1.01 to 1.5, alternatively 1.01 to 1.3, and alternatively 1.01 to 1.2.

Steps i) and ii) in the method described above may be performed with or without heating. The temperature for the reaction depends on the selection of starting materials a), b), and c) and whether any of d), e), f), g), and/or h) is present, however, the temperature may range from -20°C to 150°C; alternatively 0°C to 100°C, and alternatively 20°C to 60°C at pressure of 1 atmosphere. Pressure under which the method is performed is not critical.

The method described above may be peformed in batch, semi-batch, semi-continuous, or continuous mode in any convenient equipment. When preparing higher molecular weight copolymers (e.g., when higher molecular weight starting materials are used), the method may be performed in an extruder, such as a twin screw extruder.

The polyurethane - polyorganosiloxane copolymer described above is useful in personal care compositions. The compositions are suitable for application to various substrates including skin or hair, e.g., human skin or human hair. The polyurethane - polyorganosiloxane copolymer may act as a film forming agent in such compositions. The composition comprises (A) the polyurethane - polyorganosiloxane copolymer described above and (B) a carrier that permits application to the substrate.

Suitable carriers for personal care applications, such as skin care, include nonaqueous media capable of evaporating on contact with the skin in less than one hour, at room temperature and atmospheric pressure, e.g., isoparaffins such as isododecane and silicone oils such as caprylyl methicone.

Alternatively, the carrier may comprise a surfactant and water, e.g., the composition may form an emulsion comprising (1) the polyurethane - polyorganosiloxane copolymer, (2) a surfactant, and (3) water. As used herein, "emulsion" is meant to encompass water continuous emulsions (for example an oil in water type emulsion (o/w), or a silicone in water emulsion (s/w)), oil or silicone continuous emulsions (water in oil emulsions (w/o) or water in silicone emulsions (w/s)), or multiple emulsions (water/oil/water, oil/water/oil types, water/silicone/water, or silicone/water/silicone). The polyurethane - polyorganosiloxane copolymer may be added to any type of emulsion by common mixing techniques. The addition of the polyurethane - polyorganosiloxane copolymer may occur either during the preparation of the emulsion, or subsequently post added to a pre-formed emulsion. There are no special requirements or conditions needed to effect the mixing of polyurethane - polyorganosiloxane copolymer of the present disclosure and the emulsion. Mixing techniques can be simple stirring, homogenizing, sonolating, and other mixing techniques known in the art to effect the formation of emulsions. The mixing can be conducted in a batch, semi-continuous, or continuous process.

The amount of polyurethane - polyorganosiloxane copolymer added to the emulsion can vary and is not limited, however the amounts may range from a polyurethane - polyorganosiloxane copolymer /emulsion weight ratio of 0.1/99 to 99/0.1, alternatively 1/99 to 99/1.

The emulsions used may be w/o, w/s, or multiple phase emulsions using silicone emulsifiers. In one embodiment, the water-in-silicone emulsifier in such formulation is non-ionic and is selected from polyoxyalkylene-substituted silicones, silicone alkanolamides, silicone esters and silicone glycosides. Silicone-based surfactants may be used to form such emulsions and have been described, for example, in U.S. Patent 4,122,029 to Gee et al., U.S. Patent 5,387,417 to Rentsch, and U.S. Patent 5,811,487 to Schulz et al.

Alternatively, the emulsion containing the polyurethane - polyorganosiloxane copolymer may contain anionic surfactants, cationic surfactants, amphoteric surfactants, and nonionic surfactants. The anionic surfactants include (i) sulfonic acids and their salt derivatives, including alkyl, aralkyl, alkylnaphthalene, alkyldiphenyl ether sulfonic acids, and their salts, having at least 6 carbon atoms in the alkyl substituent, such as dodecylbenzene sulfonic acid, and its sodium salt or its amine salt; (ii) alkyl sulfates having at least 6 carbon atoms in the alkyl substituent, such as sodium lauryl sulfate; (iii) the sulfate esters of polyoxyethylene monoalkyl ethers; (iv) long chain carboxylic acid surfactants and their salts, such as lauric acid, steric acid, oleic acid, and their alkali metal and amine salts. Some other examples of anionic surfactants are alkali metal sulfosuccinates; sulfonated glyceryl esters of fatty acids such as sulfonated monoglycerides of coconut oil acids; salts of sulfonated monovalent alcohol esters such as sodium oleyl isothionate; amides of amino sulfonic acids such as the sodium salt of oleyl methyl tauride; sulfonated products of fatty acid nitriles such as palmitonitrile sulfonate; sulfonated aromatic hydrocarbons such as sodium alpha-naphthalene monosulfonate; condensation products of naphthalene sulfonic acids with formaldehyde; sodium octahydro anthracene sulfonate; alkali metal alkyl sulfates; ether sulfates having alkyl groups of eight or more carbon atoms such as sodium lauryl ether sulfate; and alkylaryl sulfonates having one or more alkyl groups of eight or more carbon atoms such as neutral salts of hexadecylbenzene sulfonic acid and C₂₀ alkylbenzene sulfonic acid.

Commercial anionic surfactants which can be used include the sodium salt of dodecylbenzene sulfonic acid sold under the trademark SIPONATE^{®} DS-10 by Alcolac Inc., Baltimore, Maryland; sodium n-hexadecyl diphenyloxide disulfonate sold under the trademark DOWFAX^{®} 8390 by The Dow Chemical Company, Midland, Michigan; the sodium salt of a secondary alkane sulfonate sold under the trademark HOSTAPUR^{®} SAS 60 by Clariant Corporation, Charlotte, North Carolina; N-acyl taurates such as sodium N-lauroyl methyl taurate sold under the trademark NIKKOL LMT^{®} by Nikko Chemicals Company, Ltd., Tokyo, Japan; and linear alkyl benzene sulfonic acids sold under the trademark BIO-SOFT^{®} S-100 by the Stepan Company, Northfield, Illinois. Compositions of the latter type such as dodecylbenzene sulfonic acid, although a catalyst as noted above, can also function as the anionic surfactant when neutralized. Other suitable surfactants include sodium alkyl sulfonate such as HOSTAPUR^{®} SAS-30. In one embodiment, the emulsifier is triethanolamine dodecylbenzene sulfonate, such as BIO-SOFT^{®} N 300.

Cationic surfactants useful herein include compounds containing quaternary ammonium hydrophilic moieties in the molecule which are positively charged, such as quaternary ammonium salts represented by R⁸R⁹R¹⁰R¹¹N⁺X⁻ where R⁸ to R¹¹ are alkyl groups containing 1-30 carbon atoms, or alkyl groups derived from tallow, coconut oil, or soy; and X is a halogen, e.g., chlorine or bromine. Alternatively, the quaternary ammonium compounds may be alkyl trimethylammonium and dialkyldimethylammonium halides, or acetates, or hydroxides, having at least 8 carbon atoms in each alkyl substituent. Dialkyl dimethyl ammonium salts can be used and are represented by R¹²R¹³N⁺(CH₃)₂X⁻ where R¹² and R¹³ are alkyl groups containing 12-30 carbon atoms or alkyl groups derived from tallow, coconut oil, or soy; and X is halogen. Monoalkyl trimethyl ammonium salts can be used and are represented by R¹⁴N⁺(CH₃)₃X⁻ where R¹⁴ is an alkyl group containing 12-30 carbon atoms or an alkyl group derived from tallow, coconut oil, or soy; and X is halogen, acetate, or hydroxide.

Representative quaternary ammonium halide salts are dodecyltrimethyl ammonium chloride/lauryltrimethyl ammonium chloride (LTAC), cetyltrimethyl ammonium chloride (CTAC), didodecyldimethyl ammonium bromide, dihexadecyldimethyl ammonium chloride, dihexadecyldimethyl ammonium bromide, dioctadecyldimethyl ammonium chloride, dieicosyldimethyl ammonium chloride, didocosyldimethyl ammonium chloride, dicoconutdimethyl ammonium chloride, ditallowdimethyl ammonium chloride, and ditallowdimethyl ammonium bromide. These quaternary ammonium salts are commercially available under trademarks such as ADOGEN^{®}, ARQUAD^{®}, TOMAH^{®}, and VARIQUAT^{®}.

Other suitable cationic surfactants which can be used include (i) fatty acid amines and amides and their salts and derivatives, such as aliphatic fatty amines and their derivatives. Such cationic surfactants that are commercially available include compositions sold under the names Arquad T27 W, Arquad 16-29, by Akzo Nobel Chemicals Inc., Chicago, Illinois; and Ammonyx Cetac-30 by the Stepan Company, Northfield, Illinois.

Suitable amphoteric surfactants include; betaines such as cocamidopropylbetaine, sultaines such as cocamidopropylhydroxysultaine, lecithin and hydrogenated lecithin, In one embodiment, the emulsifier is a combination of an anionic and nonionic surfactant. In a further embodiment, the anionic surfactant in the combination is an alkyl sulfonate or a dodecylbenzene sulfonate. In a further embodiment, the nonionic emulsifier is an alkyl-oxo alcohol polyglycol ether or an alkyl polyethylene glycol ether.

Some suitable nonionic surfactants which can be used include polyoxyethylene alkyl ethers, polyoxyethylene alkyl phenyl ethers, alkylglucosides, polyoxyethylene fatty acid esters, sorbitan fatty acid esters, and polyoxyethylene sorbitan fatty acid esters. Nonionic surfactants which are commercially available include compositions such as (i) 2,6,8-trimethyl-4-nonyl polyoxyethylene ether sold under the names Tergitol TMN-6 and Tergitol TMN-10; (ii) the C11-15 secondary alkyl polyoxyethylene ethers sold under the names Tergitol 15-S-7, Tergitol 15-S-9, Tergitol 15-S-15, Tergitol 15-S-30, and Tergitol 15-S-40, by the Dow Chemical Company, Midland, Michigan; octylphenyl polyoxyethylene (40) ether sold under the name Triton X405 by the Dow Chemical Company, Midland, Michigan; (iii) nonylphenyl polyoxyethylene (10) ether sold under the name Makon 10 by the Stepan Company, Northfield, Illinois; (iv) ethoxylated alcohols sold under the name Trycol 5953 by Henkel Corp./Emery Group, Cincinnati, Ohio; (v) ethoxylated alcohols sold under the name Brij L23 and Brij L4 by Croda Inc. Edison, NJ, (vi) alkyl-oxo alcohol polyglycol ethers such as ^{®}GENAPOL UD 050, and Genapol UD110, (vii) alkyl polyethylene glycol ether based on C10-Guerbet alcohol and ethylene oxide such as LUTENSOL^{®} XP 79.

Suitable nonionic surfactants also include poly(oxyethylene)-poly(oxypropylene)-poly(oxyethylene) tri-block copolymers. Poly(oxyethylene)-poly(oxypropylene)-poly(oxyethylene) tri-block copolymers are also commonly known as *Poloxamers.* They are nonionic triblock copolymers composed of a central hydrophobic chain of polyoxypropylene (poly(propylene oxide)) flanked by two hydrophilic chains of polyoxyethylene (poly(ethylene oxide)). Poly(oxyethylene)-poly(oxypropylene)-poly(oxyethylene) tri-block copolymers are commercially available from BASF (Florham Park, NJ) and are sold under the tradename PLURONIC^{®}, such as Pluronic L61, L62, L64, L81, P84.

The nonionic surfactant may also be a silicone polyether (SPE). The silicone polyether as an emulsifier may have a rake type structure wherein the polyoxyethylene or polyoxyethylene-polyoxypropylene copolymeric units are grafted onto the siloxane backbone, or the SPE can have an ABA block copolymeric structure wherein A represents the polyether portion and B the siloxane portion of an ABA structure. Suitable silicone polyethers include Dow Corning^{®} 5329 from Dow Corning Corporation of Midland, MI USA.

Other useful commercial nonionic surfactants are nonylphenoxy polyethoxy ethanol (10EO) sold under the trademark MAKON^{®} 10 by Stepan Company, Northfield, Illinois; polyoxyethylene 23 lauryl ether (Laureth-23) sold commercially under the trademark BRIJ^{®} 35L by ICI Surfactants, Wilmington, Delaware; and RENEX^{®} 30, a polyoxyethylene ether alcohol sold by ICI Surfactants, Wilmington, Delaware.

Protective colloids, i.e., colloidal stabilizers, may be used, if desired, to enhance stability or to provide a specific rheological characteristic to the emulsion. As used herein, the terms "protective colloid" and/or "colloidal stabilizer" mean a nonionic molecule that is an effective agent for protecting charged colloidal particles in an aqueous media against flocculation. These compositions typically have a weight average molecular weight ranging from 1,000-300,000 and are typically more hydrophilic than the composition of the first emulsion polymer, as measured by weight-averaged solubility parameters. Colloidal stabilizers which can be used include hydroxyethyl cellulose having a weight average molecular weight between 50,000-150,000; N-vinyl pyrrolidone; polyvinyl alcohol having a weight average molecular weight between 10,000-200,000; partially acetylated polyvinyl alcohol; carboxymethyl cellulose; gums such as gum arabic; starches; proteins; and mixtures thereof. Preferred colloidal stabilizers are hydroxethyl cellulose and polyvinyl alcohol.

Since emulsions are susceptible to microbiological contamination a preservative can be added. Representative preservatives, which can be used include phenoxyethanol and ethylhexylglycerin; formaldehyde; 1,3-dimethylol-5,5-dimethyl hydantoin, e.g., DMDM Hydantoin; 5-bromo-5-nitro-1,3-dioxane; methyl or propyl paraben; sorbic acid; imidazolidinyl urea; and KATHON^{®} CG (5-chloro-2-methyl-4-isothiazolin-3-one); caprylyl glycol; phenoxyethanol; benzyl alcohol; and/or benzoic acid.

The emulsions may contain a polyurethane - polyorganosiloxane copolymer concentration of 1% to 70% based on the weight of the total emulsion, alternatively 2% to 60%. While emulsions containing less than 1% polyurethane - polyorganosiloxane copolymer content can be made, such emulsions may be less valuable. The surfactant may be present at 0.05% to 30% based on the weight of the total emulsion, alternatively 0.1% to 20%. Water and optional ingredients constitute the balance of the emulsion to 100%.

The composition comprising the polyurethane - polyorganosiloxane copolymer may comprise 1% to 70%, alternatively 2% to 65%, alternatively 5% to 60%, and alternatively 20% to 50% of the the polyurethane - polyorganosiloxane copolymer, based on the weight of all ingredients in the composition. The carrier may be present in an amount sufficient to form a solution, dispersion or emulsion of the polyurethane - polyorganosiloxane copolymer. In one embodiment, the balance of the composition to 100% may be the carrier. Alternatively, the composition may further comprise one or more optional ingredients in addition to the polyurethane - polyorganosiloxane copolymer and the carrier. The selection of additional ingredients depends on the end use of the composition. The composition comprising the polyurethane - polyorganosiloxane copolymer and a carrier, as described above, may be used to prepare a personal care composition, e.g., a hair care and/or a skin care composition.

Examples of additional ingredients which may be formulated into the personal care compositions in addition to the polyurethane - polyorganosiloxane copolymer composition described above include: silicones (e.g., fluids, gums, resins, elastomers, surfactants, and/or alkylmethylsilicones, and/or silicone carbinol fluids), anti-oxidants, cleansing agents, colorants (e.g., pigments and/or dyes), conditioning agents, deposition agents, electrolytes, emollients, exfoliating agents, foam boosters, fragrances, humectants, occlusive agents, pediculicides, pH control agents, pigments, preservatives, biocides, solvents (other than the carrier), stabilizers, sun-screening agents, suspending agents, tanning agents, other surfactants (e.g., other than the surfactant used when the composition is an emulsion), thickeners, vitamins, botanicals, fragrances, waxes, rheology-modifying agents, anti-dandruff, anti-acne, anti-carie, antiperspirant/deodorant actives, pharmaceutical ingredients, and wound healing-promotion agents.

The personal care compositions may be functional with respect to the portion of the body to which they are applied, cosmetic, therapeutic, or some combination thereof. Conventional examples of such personal care compositions include
antiperspirants and/or deodorants; wound management, wound protection, and/or wound care compositions; skin barriers; liquid bandages; scar and/or stretch mark treatments; skin care creams; skin care lotions; moisturizers; facial treatments such as acne or wrinkle removers; personal and/or facial cleansers; bath oils; perfumes; colognes; sachets; sunscreens; pre-shave and/or after-shave lotions; shaving soaps and/or shaving lathers; hair shampoos; hair conditioners (either leave in or rinse off); hair colorants; hair relaxants; hair styling aids such as sprays, fixatives, mousses, gels, permanents, depilatories, and/or cuticle coats; make-ups; color cosmetics; foundations; concealers; blushes; lipsticks; eyeliners; mascara; oil removers; color cosmetic removers; powders; and/or medicament creams, pastes or sprays including antiacne, dental hygienic, antibiotic, healing promotive, and/or nutritive, which may be preventative and/or therapeutic. The polyurethane - polyorganosiloxane copolymer described above may be used as a film forming agent in any of such personal care compositions.
In general the personal care compositions may be formulated with a carrier that permits application in any conventional form, including liquids, rinses, lotions, creams, pastes, gels, foams, mousses, ointments, sprays, aerosols, soaps, sticks, soft solids, solid gels, and gels. Generally, such personal care compositions can be prepared at room temperature if no solid materials at room temperature is present in the composition, using simple propeller mixers, Brookfield counter-rotating mixers, or homogenizing mixers. No special equipment or processing conditions are typically required. Depending on the type of form made, the method of preparation will be different, but conventional methods may be used.

The personal care compositions according to this invention can be used by the standard methods, such as applying them to the human body, e.g., skin or hair, using applicators, brushes, applying by hand, pouring them and/or possibly rubbing or massaging the composition onto or into the body. Removal methods, for example for color cosmetics are also well known standard methods, including washing, wiping and peeling. For use on the skin, the personal care compositions according to the present invention may be used in a conventional manner for example for conditioning the skin. An effective amount of the composition for the purpose is applied to the skin. Such effective amounts generally range from 1 mg/cm² to 3 mg/cm². Application to the skin typically includes working the composition into the skin. This method for applying to the skin comprises the steps of contacting the skin with the composition in an effective amount and then rubbing the composition into the skin. These steps can be repeated as many times as desired to achieve the desired benefit.

The use of the personal care compositions according to the invention on hair may use a conventional manner for conditioning hair. An effective amount of the composition for cleaning and/or conditioning hair is applied to the hair. Such effective amounts generally range from 0.5 g to 50 g, alternatively from 1 g to 20 g. Application to the hair typically includes working the composition through the hair such that most or all of the hair is contacted with the personal care composition. This method for cleaning and/or conditioning the hair comprises the steps of applying an effective amount of the hair care product to the hair, and then working the composition through the hair. These steps can be repeated as many times as desired to achieve the desired conditioning benefit.

Exemplary skin care compositions that can be made with the composition comprising the polyurethane - polyorganosiloxane copolymer and the carrier, described above include, a foundation composition comprising: (1) the polyurethane - polyorganosiloxane copolymer described above, (2) water, (3) a surfactant, (4) a thickener (e.g., sodium chloride), (5) an emollient (e.g., glycerin), (6) a preservative (e.g., phenoxyethanol), (7) a pigment, and (8) a silicone (e.g., a polydimethylsiloxane and/or an alkylmethylsiloxane).

Alternatively, the skin care composition comprising the polyurethane - polyorganosiloxane copolymer and the carrier, described above, may be a lipstick composition comprising: (1) the polyurethane - polyorganosiloxane copolymer described above, (2) a solvent (e.g. isododecane and/or cyclomethicone), (3) an emollient (e.g., a vegetable oil, a plant oil, a silicone, and/or an ester), (4) a wax (e.g., polyethylene, ceresin, ozokerite, synthetic, paraffin, alkyl silicones, and/or beeswax), (5) a fixative (e.g., a silicone resins such as MQ or T^{Pr} silicone resin and/or a silicone copolymer other than the polyurethane - polyorganosiloxane copolymer described above), (6) a colorant (e.g., D&Cs Red #6, FD&C Yellow #5,6 AI Lake, Iron Oxides, TiO₂, ZnO, and/or Pearls), (7) an active (e.g., tocopherol acetate, sodium hyaluronate, amino acids, panthenol, and/or ascorbyl palmitate), (8) a fillers (e.g. mica, silica, boron nitride, starch, and/or acrylate copolymer), (9) an antioxidants and/or preservative (e.g., phenoxyethanol), and (10) a plasticizer (e.g., oleyl alcohol and/or petrolatum).

Alternatively, the skin care composition comprising the polyurethane - polyorganosiloxane copolymer and the carrier, described above, may be an antiperspirant composition comprising: (1) the polyurethane - polyorganosiloxane copolymer described above, (2) a wax and/or thickener (e.g., beeswax, sodium stearate, stearyl alcohol, and/or carnauba), (3) an emollient (*e*.*g*. mineral oil, cylcomethicone, propylene glycol, and/or alcohol, (4) a fixative (e.g., silicone MQ or copolymer type resins and/or acrylate copolymers), (5) an antiperspirant active (e.g. aluminum chlorohydrate), and (6) a fragrance and/or perfume.

Alternatively, the skin care composition comprising the polyurethane - polyorganosiloxane copolymer and the carrier, described above, may be a sunscreen composition comprising: (1) the polyurethane - polyorganosiloxane copolymer described above, (2) an organic or physical sun blocker (e.g., oxybenzone, octocrylene, titanium dioxide, zinc oxide, and/or avobenzone), (3) a preservatives (e.g., phenoxyethanol), (4) an emulsifier and/or surfactant, (5) an emollient, (6) a thickener, (7) water, and (8) a moisturizer (e.g., glycerin).

Exemplary hair care compositions that can be made with the composition comprising the polyurethane - polyorganosiloxane copolymer and the carrier, described above, include a shampoo comprising: (1) the polyurethane - polyorganosiloxane copolymer described above, or the emulsion of the polyurethane - polyorganosiloxane copolymer described above, (2) water, and (3) an anionic surfactant and/or an amphoteric surfactant (e.g., sodium laureth sulfate), optionally (4) a preservative, and optionally (5) a deposition agent (e.g., a cationic deposition polymer), and optionally (6) a thickener (e.g., carbomer).

Alternatively, the hair care product may be a hair conditioner comprising: (A) the polyurethane - polyorganosiloxane copolymer described above, or the emulsion of the polyurethane - polyorganosiloxane copolymer described above, (B) water,
optionally (C) a thickener (e.g., hydroxyethyl-cellulose), (D) a fatty alcohol (e.g., Cetearyl Alcohol), optionally (E) other emulsifiers (e.g., PEG-100 Stearate & Glyceryl Stearate), optionally (F) a preservative, and optionally (G) other conditioning agents (e.g., cationic surfactants and/or cationic polymers).

Alternatively, the composition comprising the polyurethane - polyorganosiloxane copolymer and the carrier, described above, may be used as a leave-in or leave-on hair treatment composition.

The composition comprising the polyurethane - polyorganosiloxane copolymer and a carrier, described above, can be used in a variety of personal care applications. In particular, said composition may be used in the personal care compositions disclosed in U.S. Patent 6,051,216 to Barr et al.; U.S. Patent 5,919,441 to Mendolia et al.; U.S. Patent 5,981,680 to Petroff et al.; U.S. Patent Application 2010/0098648 to Yu, and WO 2004/060101 to Yu; in sunscreen compositions as disclosed in U.S. Patent 6,916,464 to Hansenne et al.; in cosmetic compositions containing film-forming resins, as disclosed in U.S. Patent Application Publication 2003/0235552 to Yu; in the cosmetic compositions as disclosed in U.S. Patent Application Publication 2003/0235553 to Lu, U.S. Patent Application Publication 2003/0072730 to Tornilhac, U.S. Patent Application Publication 2003/0170188 to Ferrari et al., EP 1,266,647 to Tornilhac, EP 1,266,648 to Ferrari, et al., EP1,266,653 to Ferrari et al., WO2003/105789 to Lu, WO2004/000247 to Lu and WO2003/106614 to Lu; as additional agents to those disclosed in WO2004/054523 to Tournilhac; in long wearing cosmetic compositions as disclosed in U.S. Patent Application Publication 2004/0180032; and in transparent or translucent care and/or make up compositions as discussed in WO 2004/054524.

The polyurethane - polyorganosiloxane copolymer is useful as a film forming agent in various applications, such as the personal care compositions described above. Alternatively, the polyurethane - polyorganosiloxane copolymer is useful in addition to, or instead of, the silicone acrylate in the personal care compositions in, for example, "A Second Generation Silicone Acrylate for use in Beauty Care Applications, IP.com Number: 000239161 Electronic Publication Date October 17, 2014. The polyurethane - polyorganosiloxane copolymer is useful as a film forming agent in addition to, or instead of, the phenylsilsequioxane resin in, for example, "Personal Care Applications for Phenylsilsesquioxane Resins," IP.com Number IPCOM000248667D, Electronic Publication Date December 22, 2016. These references disclose various color cosmetic formulations, such as foundations, blushes, lipsticks, lip glosses, mascaras, eye shadows, eyebrow gels and eyeliners; skin care compositions such as sun care formulations, antiperspirants and deodorants; and hair care formulations such as shampoos, hair conditioners (leave on or rinse off) and hair waxes.

When selecting ingredients for the personal care compositions described above, there may be overlap between types of ingredients because certain ingredients described herein may have more than one function. For example, certain silicone MQ resins may be useful as fixatives and additional film forming agents. Certain particulates such as titanium dioxide may be useful as pigments and sun blockers. When adding additional ingredients to the composition, the additional ingredients are distinct from one another.

### EXAMPLES

These examples are intended to illustrate the invention to one of ordinary skill in the art and should not be interpreted as limiting the scope of the invention set forth in the claims. All measurements and experiments were conducted at 23°C, unless indicated otherwise. Abbreviations used in the specification are in Table 1, below.

**Table 1 - Abbreviations**

| Abbreviation | Meaning |
|---|---|
| DA | Diallyl amine from TCI |
| EtAc | Ethyl acetate, from Sigma-Aldrich. Anhydrous for polymerization reaction. HPLC grade for dissolving and processing copolymers. |
| HDI | Hexamethylene diisocyanate from Acros |
| IPDI | Isophorone diisocyanate from Alfa Aesar |
| C16 | Carbinol terminated polydimethylsiloxane having MW of 920 to 924 from Gelest, Product DMS-C16 |
| FTIR | Fourier Transform Infra-Red |
| A15 | Amino propyl terminated polydimethylsiloxane with a molecular weight Mn of 3000 from Gelest, Product DMS-A15 |
| NMR | Nuclear Magnetic Resonance |
| ml | Milliliters |
| °C | Degrees Celsius |
| Mn | Number average molecular weight determined by NMR |
| NMR | Nuclear magnetic resonance |

### Reference Example 1: General Procedure for Preparing Silicone-urethane Copolymers (not claimed)

A ml 4 neck flask was placed into a temperature controlled heating block and fitted with mechanical stirrer, thermometer, dropping funnel and reflux condenser.
1) The flask was charged with an a) isocyanate compound and a b) polyorganosiloxane, which were mixed to form a mixture.
2) The mixture was stirred and heated at 60°C, and the progress of the reaction followed by FTIR.
3) An c) endblocker (and optionally e) solvent) were charged to the dropping funnel and added drop-wise to the mixture in the flask, which was then heated for a period of time.
4) The mixture in the flask was cooled to room temperature and filtered through a 0.45 micron filter using Celite^{®} 545 filter aid. The filtrate was transferred into a round flask and volatiles removed with a rotary evaporator (90°C, 1 mbar).

Copolymers were prepared according to this procedure using starting materials and conditions shown in Table 2 and labeled as HDIₙC16ₘDA₂. The copolymers were characterized, and results are in Table 4.

**Table 2 - Polyurethane - Polyorganosiloxane Copolymer Sample**

| Ingredient (grams) | 1 HDIₙC16ₘDA_{2;} (n, m) = (14, 13) |
|---|---|
| a) isocyanate compound | 40.4 g HDI |
| b) polyorganosiloxane | 211.1 g C16 |
| Time for heating in step 2) | 7 hours |
| Endblocker and optional solvent in step 3) | 200 ml EtAc and 8.4 g DA |
| Temperature for heating in step 4) | Heated at 70°C |
| Time for Heating in step 4) | 2 hours |

### Reference Example 2 - General Procedure for Preparing Silicone-urethane-urea Copolymers (not claimed)

A ml 4 neck flask was placed into a temperature controlled heating block and fitted with mechanical stirrer, thermometer, dropping funnel and reflux condenser.
1) The flask was charged with an a) polyorganosiloxane and a b) solvent.
2) The c) isocyanate compound and d) solvent were added to the dropping funnel, mixed and then slowly added to the reaction flask at room temperature of 15°C to below 40°C.
3) The mixture was stirred and heated at 60°C for 1 hours.
4) An e) endblocker (and optionally e) solvent) were charged to the dropping funnel and added drop-wise to the mixture in the flask, which was then heated at 60°C for 1 hours.
5) The mixture in the flask was cooled to room temperature and filtered through a 0.45 micron filter using Celite^{®} 545 filter aid. The filtrate was transferred into a round flask and volatiles removed with a rotary evaporator (90°C, 1 mbar).

Samples were prepared according to this procedure using starting materials and conditions shown in Table 3 and labeled as IPDIₙA15ₘDA₂.

**Table 3 - Polyurethane - Polyorganosiloxane Copolymer Sample**

| Ingredient (grams) | Sample 2 IPDIₙA15ₘDA₂, (n, m) = (10, 9) |
|---|---|
| a) polyorqanosiloxane | 81.7 q A15 |
| b) Solvent | 150 ml EtAc |
| c) isocyanate compound | 6.7 g IPDI |
| d) Solvent | 150 ml EtAc |
| Endblocker and optional solvent in step 4) | 2.4 q DA and 150 ml EtAc |

**Table 4 - Polyurethane - Polyorganosiloxane Copolymer Characterization**

| Sample/ Test | Sample 1 HDI₁₄C16₁₃DA₂ | Sample 2 IPDI₁₀A15₉DA₂ |
|---|---|---|
| Mn (g/mol) | 20700 | 29400 |
| ¹H-NMR analysis | Olefinic (5.83 - 5.73, 5.19 - 5.13 ppm), NH (4.64, 4.43), -C*H*₂OOC (3.99 - 3.96), -C*H*₂-CH=CH₂ (3.84-3.82), *-*C*H*₂-OH (3.59 - 3.56), -C*H*₂-NHCO (3.20 - 3.11), -C*H*₂- (1.64-1.58, 1.50 - 1.45, 1.33 - 1.30),-C*H*₂-Si (0.54 - 0.49), Si-C*H*₃ (0.07-0.02) | Olefinic (5.84 - 5.75, 5.19 - 5.14 ppm), NH (4.64, 4.43), -C*H*₂OOC (3.99-3.96), -C*H*₂-CH=CH₂ (3.87- 3.81),-C*H*₂-NHCO (3.21 - 2.92), -C*H*₂- (1.70-1.48), ), -C*H*₂- (1.14 - 0.81),-C*H*₂-Si (0.54 - 0.49), Si-C*H*₃ (0.21 - 0.09) |
| ¹³C-NMR analysis | NC=ONH, NH*C*=OO (158.51, 157.15), -H*C*=CH₂ (134.69),-HC=*C*H₂ (116.88), -*C*H₂OOC (67.51), N-CH₂-HC=CH₂ (49.71),-*C*H₂NH*C*=OO *C*H₂NH*C*=ON (41.09, 40.88), -*C*H₂- (30.47, 30.31, 26.63), -*C*H₂-(23.34), Si-*C*H₂- (14.32), Si-CH₃ (1.47 - 0.42) | NC=ONH, NH*C*=OO (164.33, 156.55),-HC=*C*H₂ (135.97), -H*C*=CH₂ (116.70, N-*C*H₂-HC=CH₂ (49.35),-*C*H₂NHC=ONH, *C*H₂NHC=ON (43.56, 43.49), -*C*H₂- (31.89, 27.86, 27.68),-*C*H₂- (24.21), Si-*C*H₂- (15.14), Si-C*H*₃ (1.37 - 0.12) |

### Example 3: Foundation Composition

A foundation composition was prepared by mixing the ingredients in Table 5, as follows. The ingredients in phase A were mixed until homogenous, the ingredients in phase B were mixed until homogenous, and the ingredients in phase C were mixed using a high shear mixer to form a dispersion. Next, phase C was added to phase A. This was mixed while adding phase B slowly with a high rate of agitation.

**Table 5 - Foundation Composition using Copolymers of Reference Examples 1 and 2**

| | | | | |
|---|---|---|---|---|
| | *Copolymer Sample* | | 2 | 1 |
| Copolymer solution concentration by weiqht | *Solids content (i.e., amount of copolymer in solution):* | | **20.00%** | **50.00%** |

| **Ingredient** | **Trade Name/Supplier** | **Wt. %** | **Grams** | **Grams** |
|---|---|---|---|---|
| **Phase A** | | | | |
| Surfactant Blend | ES-5300 Formulation Aid from Dow Corning Corporation | 6 | 1.20 | 1.20 |
| Isododecane | -------- | q.s. | ----- | 1.40 |
| Film-Former (% of copolymer in the foundation composition) | -------- | 5 | 5.00 | 2.00 |

| **Phase B** | | | | |
|---|---|---|---|---|
| Water | --------- | 59.5 | 10.3 | 11.9 |
| Sodium Chloride | Fisher | 1 | 0.2 | 0.2 |
| Glycerin | Fisher | 5 | 1 | 1 |
| Phenoxyethanol, Ethylhexylglycerin | Euxyl^{®} PE 9010/Schülke & Mayr GmbH | 1 | 0.2 | 0.2 |

| **Phase C** | | | | |
|---|---|---|---|---|
| Iron Oxide (CI77499), Dimethicone | SA-C335000-10/Miyoshi Europe S.A.S. | 0.07 | 0.01 | 0.01 |
| Iron Oxide (CI77491), Dimethicone | SA-C332199-10/Miyoshi Europe S.A.S. | 0.25 | 0.05 | 0.05 |
| Iron Oxide (CI77492), Dimethicone | SA-C331700-10/Miyoshi Europe S.A.S. | 1.09 | 0.22 | 0.22 |
| Titanium Dioxide, Dimethicone | SA-TAO-77891/Miyoshi Europe S.A.S. | 5.81 | 1.16 | 1.16 |
| Caprylyl Methicone | Dow Corning^{®} FZ-3196 Volatile Alkyl Methyl Siloxane Fluid | 3.28 | 0.66 | 0.66 |
| | | *total (g):* | 20.00 | 20.00 |

### Example 4 (comparative)

Comparison samples were prepared by either taking away the copolymer and replacing it with isododecane (Control in table 6), or by replacing the copolymer with a comparative film former, which was an MQ resin produced by Dow Corning Corporation commercially being used as a film former in similar types of formulations (MQ Resin (749) in Table 6).

### Example 5 - Evaluation of Samples prepared in Example 3 and comparative Example 4.

Hydrated collagen substrate samples were prepared by pulling hydrated collagen over polycarbonate blocks. The foundation compositions described above were coated onto the hydrated collagen using a 2 micrometer film gauge. The resulting coating had a thickness of 150 micrometers. Each foundation composition was allowed to dry overnight to form a foundation on the hydrated collagen. Using a Gardner abrasion tester fitted with an adhesive strip of the loop side of Velcro ^{™}, each sample was insulted for either 50 or 20 cycles, depending on whether the film was dry or sebum was added. Three blocks were tested per foundation. Each foundation was tested dry, however some of the best performers were tested for sebum resistance which required adding a drop of artificial sebum to the foundation, and evenly spreading it out; and then waiting a minute before beginning the abrasion testing.

A Hunterlab colorimeter was used to measure the LAB (color) value for each block using 0.5" diameter filter. Measurements were taken at 0, 4, 8, 12, 16, 20, 30, 40, and 50 insults. If using sebum the testing stopped at 20 insults. From this a ΔE was calculated to determine the total change in color.

Table 6 shows abrasion resistance test results, as measured by the amount of color changes.

**Table 6**

| #of Abrasion Cycles | ΔE | | | |
|---|---|---|---|---|
| | Control | MQ Resin (749) | Sample 2 | Sample 1 |
| 0 | 0 | 0 | 0 | 0 |
| 4 | 7.94 | 3.56 | 5.26 | 0.79 |
| 8 | 9.42 | 3.79 | 6.83 | 1.19 |
| 12 | 10.28 | 3.80 | 7.90 | 1.07 |
| 16 | 10.81 | 3.99 | 9.02 | 0.98 |
| 20 | 11.11 | 4.17 | 9.88 | 1.08 |
| 30 | 11.67 | 4.52 | 11.56 | 1.65 |
| 40 | 12.00 | 4.88 | 12.54 | 2.29 |
| 50 | 12.18 | 5.06 | 12.80 | 2.90 |

Contact angle was measured on the samples by diluting the copolymers prepared in Reference Example 1 and Reference Example 2 in ethyl acetate, to form solutions that were 80% ethyl acetate and 20% copolymer. The solutions were then coated onto cleaned glass microscope slides using a 50 micrometer film gauge. Three slides were made for each sample, and the slides were allowed to dry overnight. A VCA Optima device was used to test the slides for both water and artificial sebum. For water, 2 microns of water was added to slides with each sample dried thereon. For sebum, 1 microliter of artificial sebum was added. One contact angle measurement was taken per slide for each water and artificial sebum.

Solution samples were prepared by diluting the copolymers prepared in Reference Example 1 and Reference Example 2 in ethyl acetate, to form solutions that were 80% ethyl acetate and 20% copolymer. The solution samples were coated onto 10 centimeter long latex elastic bands using a 50 micrometer film gauge. The latex bands were 0.33 millimeter thick specimens from Four D Rubber Co. Ltd. in United Kingdom, Red Latex Rubber: incolore solution. One band was coated per sample, and coated bands were allowed to dry overnight. Photos were taken the next day before any measurements. Films were then stretched to 200% of their original length (20cm). Photos were taken again to capture any cracking or differences in shine. The shine test results are summarized in Table 7.

**Table 7**

| Sample | Original (Before Stretching) | After Stretching (200% Elongation) |
|---|---|---|
| Copolymer of Reference Example 1 | Shiny | Shiny, little change |
| Copolymer of Reference Example 2 | Shiny | Shiny, some matting |
| Control | Matte | Matte, bad cracks |
| MQ Resin (749) | Shiny | Matte, cracks present |

Example 5 shows that the foundation prepared from the personal care composition of this invention, which includes a polyurethane - polyorganosiloxane copolymer, has superior abrasion resistance to a foundation prepared using a composition with a conventional MQ resin film forming agent. Additional benefits include improved flexibility for comfort of wear, and shine. Example 6: Preparation of Emulsions (Prophetic)

The emulsification procedure will be performed on a Hauschild Speedmixer. To a plastic cup will be added polyurethane - polyorganosiloxane copolymer prepared in Reference Example 1, Brig 35L surfactant, optionally lactic acid, and a first quantity of water. The mixture will be sheared at maximum speed for 20 seconds. An additional quantity of water will be added, and the emulsion will again be mixed on the Speedmixer. The amounts in each emulsion sample to be prepared are shown in Table 6, below.

**Table 6 - Ingredients for preparing emulsions, weight parts**

| Ingredient | Amount in Sample A | Amount in Sample B | Amount in Sample C | Amount in Sample D |
|---|---|---|---|---|
| polyurethane - polyorganosiloxane copolymer | 10 | 4 | 10 | 4 |
| Brig 35L Surfactant | 0.5 | 4 | 0.6 | 4 |
| Water, First Quantity + Additional Quantity | 3+3 | 2+8 | 2+4 | 2+7 |
| Lactic Acid | none | 0.1 | none | 0.06 |

### Example 7: Preparation of Hair Conditioner Formulations (Prophetic)

Samples of emulsions as described in Example 6 will be added to rinse-off conditioning formulations using an amount sufficient to provide 2% the silicone block copolymer. The conditioning formulations are shown in Table 8, below. The conditioners of the present invention will be prepared using Emulsions A, B, C and D from Table 6.

**Table 8 - Conditioners**

| Ingredient | Weight % | Weight % | Weight % | Weight % |
|---|---|---|---|---|
| Deionized Water | q.s. to 100 % | q.s. to 100 % | q.s. to 100 % | q.s. to 100 % |
| Hydroxyethyl-cellulose¹ | 1.5 | 1.5 | 1.5 | 1.5 |
| Cetearyl Alcohol² | 1.0 | 1.0 | 1.0 | 1.0 |
| PEG-100 Stearate & Glyceryl Stearate³ | 1.0 | 1.0 | 1.0 | 1.0 |
| Emulsion A | 3.3 | ---- | ---- | ---- |
| Emulsion B | ---- | 10.0 | ---- | ---- |
| Emulsion C | ---- | ---- | 3.4 | ---- |
| Emulsion D | ---- | ---- | ---- | 9.6 |
| Phenoxyethanol and Ethylhexylglycerin⁴ | 0.5 | 0.5 | 0.5 | 0.5 |

| | | | | |
|---|---|---|---|---|
| 1. Natrosol^{®} 250 HHR available from Hercules of Wilmington, DE 2. Crodocol CS-50^{®} available from Croda Inc. of Edison, NJ 3. Arlacel^{®} 165 available from Uniqema of Wilmington, DE 4. Euxyl^{®} PE 9010 available from Schülke & Mayr | | | | |

### Example 8 - Conditioning Shampoo Formulations (Prophetic)

Samples of emulsions described in Example 6 will be added to shampoo formulations in an amount sufficient to provide 2% of the polyorganosiloxane - polyurethane copolymer. The shampoo formulations are shown in Table 9. The shampoos of the present invention will be prepared using A, B, C and D emulsions from Table 6.

**Table 9 - Conditioning Shampoos**

| Ingredient | Weight % | Weight % | Weight % | Weight % |
|---|---|---|---|---|
| Deionized Water | q.s. to 100% | q.s. to 100% | q.s. to 100% | q.s. to 100% |
| Sodium Laureth Sulfate¹ | 30 | 30 | 30 | 30 |
| Cocamide MIPA² | 2.0 | 2.0 | 2.0 | 2.0 |
| Cocamidopropyl Betaine³ | 7.0 | 7.0 | 7.0 | 7.0 |
| Polyquaternium-10⁴ | 0.3 | 0.3 | 0.3 | 0.3 |
| PEG-150 Pentaerythrityl Tetrastearate⁵ | 1.5 | 1.5 | 1.5 | 1.5 |
| Emulsion A | 3.3 | ---- | ---- | ---- |
| Emulsion B | ---- | 10.0 | ---- | ---- |
| Emulsion C | ---- | ---- | 3.4 | ---- |
| Emulsion D | ---- | ---- | ---- | 9.6 |
| Phenoxyethanol (and) Ethylhexylglycerin⁶ | 0.5 | 0.5 | 0.5 | 0.5 |

| | | | | |
|---|---|---|---|---|
| 1. Standapol ES-3^{®} available from Cognis Corp. of Cincinnati, OH 2. Mackamide CPA available from Rhodia3. Monateric CAB-LC^{®} available from Uniquema of New Castle, DE 4. UCARE Polymer JR-30M available from Dow/Amerchol of Midland, MI 5. Crothix ^{®} available from Croda Inc. of Edison, NJ 6. Euxyl^{®} PE 9010 available from Schülke & Mayr | | | | |

Deionized water is added to the mixing vessel. In order to keep the active silicone loading constant throughout testing, the water level may be adjusted by adding water depending on the percent copolymer in the various emulsions used. With moderate agitation, the polyquaternium-10 is dispersed until fully dissolved. This is then heated to 75°C and the PEG-150 pentaerythrityl tetrastearate is added with continual mixing. Heat is decreased to 40°C and sodium lauryl ether sulfate, cocamide MIPA, cocamidopropyl betaine are added in that order. When completely incorporated, polyurethane - polyorganosiloxane copolymer emulsion is added to the base shampoo. The shampoo is mixed for 5-10 minutes and then Phenoxyethanol (and) Ethylhexylglycerin is added. The water loss is compensated for and the formulation is mixed for an additional 5 minutes. The final pH of the shampoo formulations are approximately 5.5-6.0.

### Reference Example 9 - General Procedure for Preparing Copolymers

A ml 4 neck flask was placed into a temperature controlled heating block and fitted with mechanical stirrer, thermometer, dropping funnel and reflux condenser.
1) The flask was charged with an a) isocyanate compound, an b) endblocker and a c) catalyst, which were mixed to form a mixture.
2) The mixture was stirred and heated at 60°C for 1 hour, and the progress of the reaction followed by FTIR.
3) After a period of time the reaction was cooled to room temperature of 15°C to 30°C and solvent added.
4) A d) polyorganosiloxane and a e) polyethylene glycol (and optionally f) solvent) were charged to the dropping funnel and added drop-wise to the mixture in the flask, which was then heated for a period of time at 60°C.
5) f) EtOH was added to the mixture in the flask, which was then heated to reflux for period of time.
6) The mixture in the flask was cooled to room temperature and filtered through a 0.45 micron filter using Celite^{®} 545 filter aid. The filtrate was transferred into a round flask and volatiles removed with a rotary evaporator (90°C, 1 mbar).

Samples were prepared according to this procedure using starting materials and conditions shown in Table 10.

**Table 10**

| Reference Example 9 | a) isocyanate compound | b) Endblocker, c) Catalyst | d) polyorganosiloxane | e) polyethylene glycol f) Solvent | Time for Heating in step 4) | f) EtOH | Time for Heating in step 5) |
|---|---|---|---|---|---|---|---|
| 10-1 HDIₙC62ₘPEG400ₒTPDA_{2;} (n, m, o) = (14, 6.5, 6.5) | 16.6 g HDI | 3.2 g TPDA 0.08 g DBTL | 79.8 g C62 | 18.7 g PEG400 100 ml EtAc | 5 hours | 5g EtOH | 2 hours |
| 10-2 MDIₙC62ₘPEG400ₒTPDA_{2;} (n, m, o) = (14.2, 6.6, 6.6) | 22.1 g MDI | 2.8 g TPDA 0.08 g DBTL | 71.6 g C62 | 16.8 g PEG400 100 ml EtAc | 4 hours | 5g EtOH | 2 hours |

**Table 11**

| Example | Molecular Weight (Mn) (g/mol) | ¹H-NMR analysis | ¹³C-NMR analysis |
|---|---|---|---|
| 10-1 | 16500 | Olefinic (5.88 - 5.80, 5.25 - 5.10 ppm), NH (4.91, 4.36), -C*H*₂OOC (4.19 - 4.17), COO-C*H*₂-C(Et)- (4.00), {Vi-C*H*₂}₂-O- (3.93- 3.90), -C*H*₂-OC*H*₂C*H*₂O-C*H*₂- -C*H*₂-O-C₃H₆-Si (3.71 - 3.58), -O-C*H*₂-C₃H₆-Si (3.42- 3.38), {Allyl-O-C*H*₂}₂- (3.29), -C*H*₂-NHCO (3.16-3.11), -C*H*₂- (1.63 - 1.55, 1.48 - 1.45, 1.32-1.29), C-CH₂-C*H*₃ (0.86 - 0.82), C-C*H*₂-Si (0.52 - 0.48), Si-C*H*₃ (0.08 - 0.03). | NH-CO-O (158.76), -H*C*=CH₂ (135.27), -H*C*=CH₂ (116.37), O-*C*H₂-C₂H₄-Si (74.14), Vi-*C*H₂-O (72.37), -(O-*C*H₂-*C*H₂)ₙ-O-(70.72), Allyl-O-*C*H₂- (69.80), - *C*H₂-(O-C₂H₄)ₙ-O-*C*H₂-(69.19), ≡C-*C*H₂-O-CO-NH- 65.09, -*C*H₂-CH₂-(O-C₂H₄)ₙ-O-CH₂-*C*H₂- (63.89), -OC-O-*C*H₂-CH₂-O-C₃H₆-Si (61.69), Et-*C*≡ (42.65), -C₄H₈-*C*H₂-NH-*C*=O-O (41.94, 41.56), -*C*H₂- (30.69, 26.47) 26.58), -*C*H₂-CH₂-O-Si (23.51), CH₃-*C*H₂-*C≡* (23.09), Si-*C*H₂- (14.23), CH₃-*C*H₂-*C≡* (7.79), Si-C*H*₃ (1.16 - 0.27). |
| 10-2 | 17900 | Aromatic (7.74 - 7.71, 7.29 - 7.20, 7.08-7.03, 6.71 - 6.68, 6.46 - 6.38), Olefinic (5.90 - 5.80, 5.25 - 5.10 ppm), -*C*H₂OOC (4.28 - 4.25, COO-C*H*₂-C(Et)- (4.12), {Vi-C*H*₂}₂-O-(3.93 - 3.92), Ph-CH₂-Ph (3.89 - 3.85),-C*H*₂-OC*H*₂C*H*₂O-C*H*₂- -C*H*₂-O-C₃H₆-Si (3.71 - 3.58), -O-C*H*₂-C₃H₆-Si (3.45- 3.41), {Allyl-O-C*H*₂}₂- (3.33), -C*H*₂-NHCO (3.16-3.11), -C*H*₂- (1.65 - 1.58), C-CH₂-C*H*₃ (1.48 - 1.42), C-CH₂-C*H*₃ (0.88 - 0.84), C-C*H*₂-Si (0.54 - 0.49 ), Si-C*H*₃ (0.08 - 0.03). | NH-CO-O (154.40, 154.02), Aromatic (137.20 -136.02), HC=*C*H₂ (135.30), Aromatic (133.97, 132.55, 132.31, 130.69, 129.49, 127.54, 125.11, 123.46, 123.35, 119.28), -H*C*=CH₂ (116.65), O-*C*H₂-C₂H₄-Si (74.31), Vi-*C*H₂-O (72.53), -(O-*C*H₂-*C*H₂)ₙ-O- (70.74), Allyl-O-*C*H₂- (69.68), - *C*H₂-(O-C₂H₄)ₙ-O-*C*H₂- (69.13), ≡C-*C*H₂-O-CO-NH- 65.70, -*C*H₂-CH₂-(O-C₂H₄)ₙ-O-CH₂-*C*H₂- -OC-O-*C*H₂-CH₂-O-C₃H₆-Si (64.62, 64.31), -OC-O-CH₂-*C*H₂-O-C₃H₆-Si (61.97, 61.78), Et-*C*≡ 42.78, -Ph-*C*H₂-Ph- (40.84, 37.27), -*C*H₂-CH₂-Si (23.65), CH₃-CH₂-*C*≡ (23.25), -*C*H₂-Si (14.38), CH₃-*C*H₂-*C≡* (7.94), Si(CH₃)₂ (1.30 - 0.4). |

All amounts, ratios, and percentages are' by weight unless otherwise indicated. The articles 'a', 'an', and 'the' each refer to one or more, unless otherwise indicated. The disclosure of ranges includes the range itself and also anything subsumed therein, as well as endpoints. For example, disclosure of a range of 2.0 to 4.0 includes not only the range of 2.0 to 4.0, but also 2.1, 2.3, 3.4, 3.5, and 4.0 individually, as well as any other number subsumed in the range. Furthermore, disclosure of a range of, for example, 2.0 to 4.0 includes the subsets of, for example, 2.1 to 3.5, 2.3 to 3.4, 2.6 to 3.7, and 3.8 to 4.0, as well as any other subset subsumed in the range. Similarly, the disclosure of Markush groups includes the entire group and also any individual members and subgroups subsumed therein. For example, disclosure of the Markush group a hydrogen atom, an alkyl group, an alkenyl group, or an aryl group, includes the member alkyl individually; the subgroup alkyl and aryl; and any other individual member and subgroup subsumed therein.

"Alkyl" means a saturated monovalent hydrocarbon group. Alkyl is exemplified by methyl, ethyl, propyl *(e.g.,* iso-propyl and/or n-propyl), butyl *(e.g.,* isobutyl, n-butyl, tert-butyl, and/or sec-butyl), pentyl (e.g., isopentyl, neopentyl, and/or tert-pentyl); hexyl, heptyl, octyl, nonyl, and decyl, as well as branched saturated monovalent hydrocarbon groups of 6 or more carbon atoms.

"Alkenyl" means a monovalent hydrocarbon group containing a double bond. Alkenyl groups are exemplified by ethenyl, propenyl (e.g., iso-propenyl and/or n-propenyl), butenyl *(e.g.,* isobutenyl, n-butenyl, tert-butenyl, and/or sec-butenyl), pentenyl (*e.g.,* isopentenyl, n-pentenyl, and/or tert-pentenyl), hexenyl, heptenyl, octenyl, nonenyl, and decenyl, as well as such branched groups of 6 or more carbon atoms.

"Alkynyl" means a monovalent hydrocarbon group containing a triple bond. Alkynyl groups are exemplified by ethynyl, propynyl (e.g., iso-propynyl and/or n-propynyl), butynyl *(e.g.,* isobutynyl, n-butynyl, tert-butynyl, and/or sec-butynyl), pentynyl *(e.g.,* isopentynyl, n-pentynyl, and/or tert-pentynyl), hexynyl, heptynyl, octynyl, nonynyl, and decynyl, as well as such branched groups of 6 or more carbon atoms.

"Aryl" means a cyclic, fully unsaturated, hydrocarbon group. Aryl is exemplified by, but not limited to, cyclopentadienyl, phenyl, anthracenyl, and naphthyl. Monocyclic aryl groups may have 5 to 9 carbon atoms, alternatively 6 to 7 carbon atoms, and alternatively 5 to 6 carbon atoms. Polycyclic aryl groups may have 10 to 18 carbon atoms, alternatively 10 to 14 carbon atoms, and alternatively 12 to 14 carbon atoms.

"Aralkyl" means an alkyl group having a pendant and/or terminal aryl group or an aryl group having a pendant alkyl group. Exemplary aralkyl groups include tolyl, xylyl, benzyl, phenylethyl, phenyl propyl, and phenyl butyl.

"Carbocycle" and "carbocyclic" each mean a hydrocarbon ring. Carbocycles may be monocyclic or alternatively may be fused, bridged, or spiro polycyclic rings. Monocyclic carbocycles may have 3 to 9 carbon atoms, alternatively 4 to 7 carbon atoms, and alternatively 5 to 6 carbon atoms. Polycyclic carbocycles may have 7 to 18 carbon atoms, alternatively 7 to 14 carbon atoms, and alternatively 9 to 10 carbon atoms. Carbocycles may be saturated or partially unsaturated.

"Cycloalkyl" means saturated carbocycle. Monocyclic cycloalkyl groups are exemplified by cyclobutyl, cyclopentyl, and cyclohexyl.

Collectively, the term "monovalent hydrocarbon group" includes alkyl, alkenyl, aryl, aralkyl, and carbocyclic groups, as defined above.

"Divalent hydrocarbon group" includes alkylene groups such as ethylene, propylene (including isopropylene and n-propylene), and butylene (including n-butylene, t-butylene and isobutylene); and pentylene, hexylene, heptylene, octylene, and branched and linear isomers thereof; arylene groups such as phenylene; and alkaralkylene groups such as: 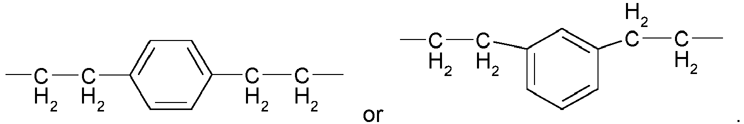 Alternatively, each divalent hydrocarbon group may be ethylene, propylene, butylene or hexylene. Alternatively, each divalent hydrocarbon group may be ethylene or propylene.

"Halogenated hydrocarbon" means a hydrocarbon group as defined above, but where one or more hydrogen atoms bonded to a carbon atom have been formally replaced with a halogen atom. For example, monovalent halogenated hydrocarbon groups can be any one of alkyl, alkenyl, aryl, aralkyl, and carbocyclic groups in which one or more hydrogen atoms bonded to a carbon atom have been replaced with a halogen atom. Monovalent halogenated hydrocarbon groups include haloalkyl groups, halogenated carbocyclic groups, and haloalkenyl groups. Haloalkyl groups include fluorinated alkyl groups such as trifluoromethyl (CF₃), fluoromethyl, trifluoroethyl, 2-fluoropropyl, 3,3,3-trifluoropropyl, 4,4,4-trifluorobutyl, 4,4,4,3,3-pentafluorobutyl, 5,5,5,4,4,3,3-heptafluoropentyl, 6,6,6,5,5,4,4,3,3-nonafluorohexyl, and 8,8,8,7,7-pentafluorooctyl; and chlorinated alkyl groups such as chloromethyl and 3-chloropropyl. Halogenated carbocyclic groups include fluorinated cycloalkyl groups such as 2,2-difluorocyclopropyl, 2,3-difluorocyclobutyl, 3,4-difluorocyclohexyl, and 3,4-difluoro-5-methylcycloheptyl; and chlorinated cycloalkyl groups such as 2,2-dichlorocyclopropyl, 2,3-dichlorocyclopentyl. Haloalkenyl groups include chloro allyl.

"Skin" includes stratum corneum covered skin and mucosal membranes.

## Claims

1. A personal care composition comprising:
(1) a polyurethane - polyorganosiloxane copolymer of the formula (I): 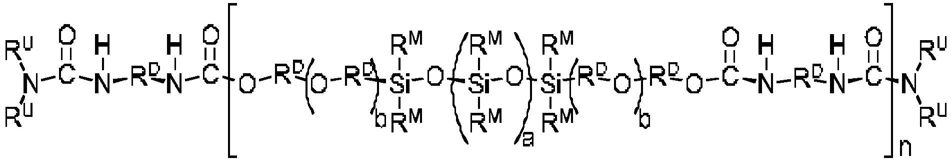 where each R^{U} is independently an alkenyl group of 2 to 13 carbon atoms; each R^{D} is independently an alkylene group of 2 to 13 carbon atoms; each R^{M} is independently a monovalent hydrocarbon group or a monovalent halogenated hydrocarbon group; each subscript b is independently 0 or 1; each subscript a is independently 0 to 100,000; and subscript n is 1 to 10,000; and
(2) a carrier that permits application.

2. The composition of claim 1, where the composition is selected from a solution or a dispersion.

3. The composition of claim 1, where the composition is an emulsion, and the carrier comprises water and a surfactant.

4. A method comprising:
(1) applying the composition of any one of claims 1 to 3 to a substrate, and
(2) drying the substrate.

5. The method of claim 4, where the substrate is selected from the group consisting of human skin and human hair.

6. Use of a polyurethane - polyorganosiloxane copolymer as a film forming agent, where the polyurethane - polyorganosiloxane copolymer has the formula (I): 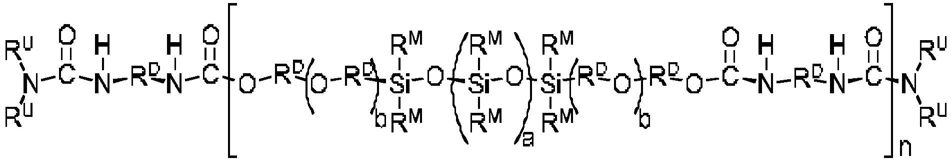 where each R^{U} is independently an alkenyl group of 2 to 13 carbon atoms; each R^{D} is independently an alkylene group of 2 to 13 carbon atoms; each R^{M} is independently a monovalent hydrocarbon group or a monovalent halogenated hydrocarbon group; each subscript b is independently 0 or 1; each subscript a is independently 0 to 100,000; and subscript n is 1 to 10,000.

7. A personal care composition comprising:
(1) a polyurethane - polyorganosiloxane copolymer of formula (II) 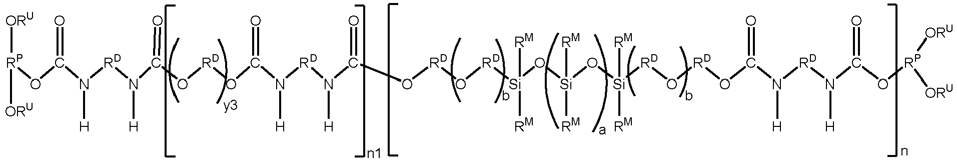 where
each R^{U} is independently an alkenyl group of 2 to 13 carbon atoms;
each R^{D} is independently an alkylene group of 2 to 13 carbon atoms;
each R^{M} is independently a monovalent hydrocarbon group or a monovalent halogenated hydrocarbon group;
each R^{P} is independently a trivalent hydrocarbon group or a trivalent halogenated hydrocarbon group;
each subscript b is independently 0 or 1;
each subscript a is independently 0 to 100,000; and
subscript n is 1 to 10,000
each subscript y3 is independently 1 to 200,000; and
subscript n1 is 1 to 1,500,000.

8. The composition of claim 7, where the composition is selected from a solution or a dispersion.

9. The composition of claim 8, where the composition is an emulsion, and the carrier comprises water and a surfactant.

10. A method comprising:
(1) applying the composition of any one of claims 7 to 9 to a substrate, and
(2) drying the substrate.

11. The method of claim 10, where the substrate is selected from the group consisting of human skin and human hair.

12. Use of a polyurethane - polyorganosiloxane copolymer as a film forming agent, where the polyurethane - polyorganosiloxane copolymer has formula (II): 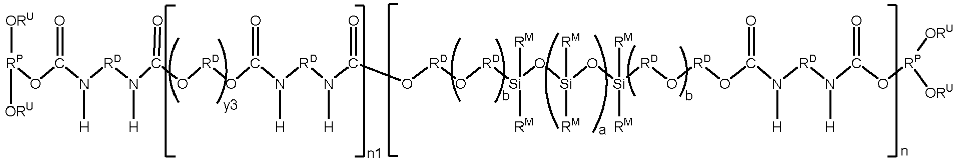 where
each R^{U} is independently an alkenyl group of 2 to 13 carbon atoms;
each R^{D} is independently an alkylene group of 2 to 13 carbon atoms;
each R^{M} is independently a monovalent hydrocarbon group or a monovalent halogenated hydrocarbon group;
each R^{P} is independently a trivalent hydrocarbon group or a trivalent halogenated hydrocarbon group ;
each subscript b is independently 0 or 1;
each subscript a is independently 0 to 100,000; and subscript n is 1 to 10,000
each subscript y3 is independently 1 to 200,000; and subscript n1 is 1 to 1,500,000.

## Patentansprüche

1. Eine Körperpflegezusammensetzung, die Folgendes beinhaltet:
(1) ein Polyurethan-Polyorganosiloxan-Copolymerder Formel (I): 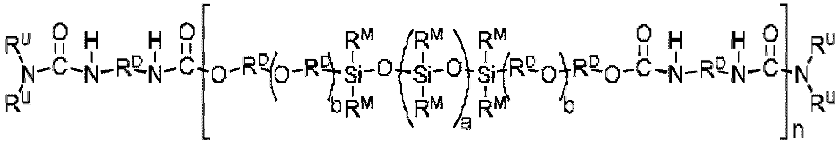 wobei jedes R^{U} unabhängig eine Alkenylgruppe von 2 bis 13 Kohlenstoffatomen ist; jedes R^{D} unabhängig eine Alkylengruppe von 2 bis 13 Kohlenstoffatomen ist; jedes R^{M} unabhängig eine monovalente Kohlenwasserstoffgruppe oder eine monovalente halogenierte Kohlenwasserstoffgruppe ist; jedes tiefgestellte Zeichen b unabhängig 0 oder 1 ist; jedes tiefgestellte Zeichen a unabhängig 0 bis 100 000 ist; und das tiefgestellte Zeichen n 1 bis 10 000 ist; und
(2) einen Träger, der das Aufbringen erlaubt.

2. Zusammensetzung gemäß Anspruch 1, wobei die Zusammensetzung aus einer Lösung oder einer Dispersion ausgewählt ist.

3. Zusammensetzung gemäß Anspruch 1, wobei die Zusammensetzung eine Emulsion ist und der Träger Wasser und ein Tensid beinhaltet.

4. Ein Verfahren, das Folgendes beinhaltet:
(1) Aufbringen der Zusammensetzung gemäß einem der Ansprüche 1 bis 3 auf ein Substrat, und
(2) Trocknen des Substrats.

5. Verfahren gemäß Anspruch 4, wobei das Substrat aus der Gruppe ausgewählt ist, die aus menschlicher Haut und menschlichem Haar besteht.

6. Verwendung eines Polyurethan-Polyorganosiloxan-Copolymers als ein filmbildendes Mittel, wobei das Polyurethan-Polyorganosiloxan-Copolymer die Formel (I) aufweist: 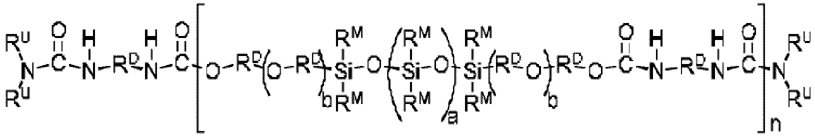 wobei jedes R^{U} unabhängig eine Alkenylgruppe von 2 bis 13 Kohlenstoffatomen ist; jedes R^{D} unabhängig eine Alkylengruppe von 2 bis 13 Kohlenstoffatomen ist; jedes R^{M} unabhängig eine monovalente Kohlenwasserstoffgruppe oder eine monovalente halogenierte Kohlenwasserstoffgruppe ist; jedes tiefgestellte Zeichen b unabhängig 0 oder 1 ist; jedes tiefgestellte Zeichen a unabhängig 0 bis 100.000 ist; und das tiefgestellte Zeichen n 1 bis 10 000 ist.

7. Eine Körperpflegezusammensetzung, die Folgendes beinhaltet:
(1) ein Polyurethan-Polyorganosiloxan-Copolymer der Formel (II) 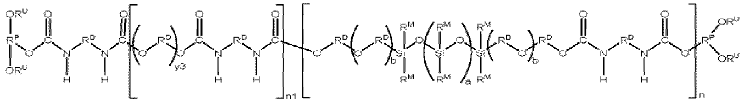 wobei
jedes R^{U} unabhängig eine Alkenylgruppe von 2 bis 13 Kohlenstoffatomen ist;
jedes R^{D} unabhängig eine Alkylengruppe von 2 bis 13 Kohlenstoffatomen ist;
jedes R^{M} unabhängig eine monovalente Kohlenwasserstoffgruppe oder eine monovalente halogenierte Kohlenwasserstoffgruppe ist;
jedes R^{P} unabhängig eine trivalente Kohlenwasserstoffgruppe oder eine trivalente halogenierte Kohlenwasserstoffgruppe ist;
jedes tiefgestellte Zeichen b unabhängig 0 oder 1 ist;
jedes tiefgestellte Zeichen a unabhängig 0 bis 100 000 ist; und
das tiefgestellte Zeichen n 1 bis 10 000 ist
jedes tiefgestellte Zeichen y3 unabhängig 1 bis 200 000 ist; und
das tiefgestellte Zeichen n1 1 bis 1 500 000 ist.

8. Zusammensetzung gemäß Anspruch 7, wobei die Zusammensetzung aus einer Lösung oder einer Dispersion ausgewählt ist.

9. Zusammensetzung gemäß Anspruch 8, wobei die Zusammensetzung eine Emulsion ist und der Träger Wasser und ein Tensid beinhaltet.

10. Ein Verfahren, das Folgendes beinhaltet:
(1) Aufbringen der Zusammensetzung gemäß einem der Ansprüche 7 bis 9 auf ein Substrat, und
(2) Trocknen des Substrats.

11. Verfahren gemäß Anspruch 10, wobei das Substrat aus der Gruppe ausgewählt ist, die aus menschlicher Haut und menschlichem Haar besteht.

12. Verwendung eines Polyurethan-Polyorganosiloxan-Copolymers als ein filmbildendes Mittel, wobei das Polyurethan-Polyorganosiloxan-Copolymer die Formel (II) aufweist: 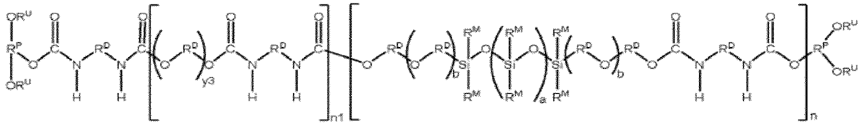 wobei
jedes R^{U} unabhängig eine Alkenylgruppe von 2 bis 13 Kohlenstoffatomen ist;
jedes R^{D} unabhängig eine Alkylengruppe von 2 bis 13 Kohlenstoffatomen ist;
jedes R^{M} unabhängig eine monovalente Kohlenwasserstoffgruppe oder eine monovalente halogenierte Kohlenwasserstoffgruppe ist;
jedes R^{P} unabhängig eine trivalente Kohlenwasserstoffgruppe oder eine trivalente halogenierte Kohlenwasserstoffgruppe ist;
jedes tiefgestellte Zeichen b unabhängig 0 oder 1 ist;
jedes tiefgestellte Zeichen a unabhängig 0 bis 100 000 ist; und
das tiefgestellte Zeichen n 1 bis 10 000 ist
jedes tiefgestellte Zeichen y3 unabhängig 1 bis 200 000 ist; und
das tiefgestellte Zeichen n1 1 bis 1 500 000 ist.

## Revendications

1. Une composition pour soins personnels comprenant :
(1) un copolymère de polyuréthane-polyorganosiloxane de la formule (I) : 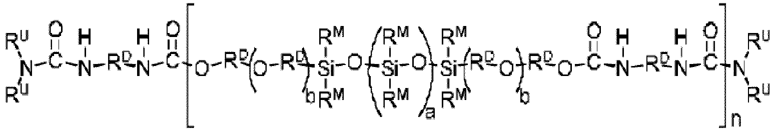 où chaque R^{U} est indépendamment un groupe alcényle de 2 à 13 atomes de carbone ; chaque R^{D} est indépendamment un groupe alkylène de 2 à 13 atomes de carbone ; chaque R^{M} est indépendamment un groupe hydrocarbure monovalent ou un groupe hydrocarbure halogéné monovalent ; chaque indice b vaut indépendamment 0 ou 1 ; chaque indice a vaut indépendamment 0 à 100 000 ; et l'indice n vaut 1 à 10 000 ; et
(2) un support qui permet l'application.

2. La composition de la revendication 1, où la composition est sélectionnée parmi une solution ou une dispersion.

3. La composition de la revendication 1, où la composition est une émulsion, et le support comprend de l'eau et un tensioactif.

4. Un procédé comprenant :
(1) l'application de la composition de n'importe laquelle des revendications 1 à 3 sur un substrat, et
(2) le séchage du substrat.

5. Le procédé de la revendication 4, où le substrat est sélectionné dans le groupe constitué de peau humaine et de cheveux humains.

6. Utilisation d'un copolymère de polyuréthane-polyorganosiloxane en tant qu'agent filmogène, où le copolymère de polyuréthane-polyorganosiloxane a la formule (I) : 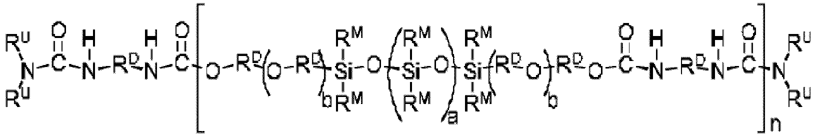 où chaque R^{U} est indépendamment un groupe alcényle de 2 à 13 atomes de carbone ; chaque R^{D} est indépendamment un groupe alkylène de 2 à 13 atomes de carbone ; chaque R^{M} est indépendamment un groupe hydrocarbure monovalent ou un groupe hydrocarbure halogéné monovalent ; chaque indice b vaut indépendamment 0 ou 1 ; chaque indice a vaut indépendamment 0 à 100 000 ; et l'indice n vaut 1 à 10 000.

7. Une composition pour soins personnels comprenant :
(1) un copolymère de polyuréthane-polyorganosiloxane de formule (II) 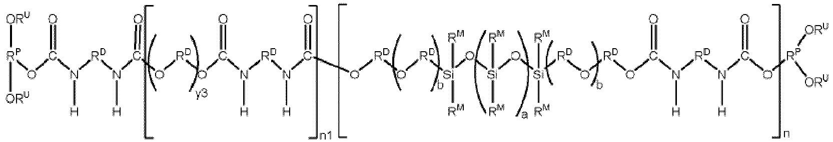 où
chaque R^{U} est indépendamment un groupe alcényle de 2 à 13 atomes de carbone ;
chaque R^{D} est indépendamment un groupe alkylène de 2 à 13 atomes de carbone ;
chaque R^{M} est indépendamment un groupe hydrocarbure monovalent ou un groupe hydrocarbure halogéné monovalent ;
chaque R^{P} est indépendamment un groupe hydrocarbure trivalent ou un groupe hydrocarbure halogéné trivalent ;
chaque indice b vaut indépendamment 0 ou 1 ;
chaque indice a vaut indépendamment 0 à 100 000 ; et
l'indice n vaut 1 à 10 000
chaque indice y3 vaut indépendamment 1 à 200 000 ; et
l'indice n1 vaut 1 à 1 500 000.

8. La composition de la revendication 7, où la composition est sélectionnée parmi une solution ou une dispersion.

9. La composition de la revendication 8, où la composition est une émulsion, et le support comprend de l'eau et un tensioactif.

10. Un procédé comprenant :
(1) l'application de la composition de n'importe laquelle des revendications 7 à 9 sur un substrat, et
(2) le séchage du substrat.

11. Le procédé de la revendication 10, où le substrat est sélectionné dans le groupe constitué de peau humaine et de cheveux humains.

12. Utilisation d'un copolymère de polyuréthane-polyorganosiloxane en tant qu'agent filmogène, où le copolymère de polyuréthane-polyorganosiloxane a la formule (II) : 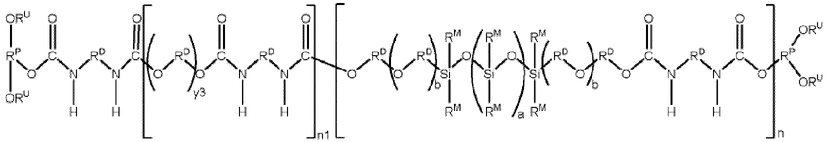 où
chaque R^{U} est indépendamment un groupe alcényle de 2 à 13 atomes de carbone ;
chaque R^{D} est indépendamment un groupe alkylène de 2 à 13 atomes de carbone ;
chaque R^{M} est indépendamment un groupe hydrocarbure monovalent ou un groupe hydrocarbure halogéné monovalent ;
chaque R^{P} est indépendamment un groupe hydrocarbure trivalent ou un groupe hydrocarbure halogéné trivalent ;
chaque indice b vaut indépendamment 0 ou 1 ;
chaque indice a vaut indépendamment 0 à 100 000 ; et
l'indice n vaut 1 à 10 000
chaque indice y3 vaut indépendamment 1 à 200 000 ; et
l'indice n1 vaut 1 à 1 500 000.
